# EUROPEAN PATENT SPECIFICATION

(11) **EP 3 727 297 B1**
(45) Date of publication and mention of the grant of the patent: **19.01.2022**
(21) Application number: 18811485.4
(22) Date of filing: 26.11.2018
(51) Int. Cl.: A61K 8/27, A61K 8/49, A61Q 17/00, A61Q 5/00

(54) **AN ANTIMICROBIAL COMPOSITION**
ANTIMIKROBIELLE ZUSAMMENSETZUNG
COMPOSITION ANTIMICROBIENNE

(30) Priority: 20.12.2017 EP 17208928
(43) Date of publication of application: 28.10.2020
(73) Proprietor: Unilever Global IP Limited, Wirral, CH62 AZD (GB); Unilever IP Holdings B.V., 3013 AL Rotterdam (NL)
(72) Inventor: DAS, Somnath, Whitefield, Bangalore 560 066 (IN); PRAMANIK, Amitava, Whitefield, Bangalore 560 066 (IN)
(74) Representative: Potter Clarkson
(86) International application number: PCT/EP2018/082591
(87) International publication number: WO 2019/120893

(56) References cited:
- US-A1- 2004 191 331
- Satyawati S Joshi: "Crystal Habit Modification using Habit Modifiers", , 2 July 2012 (2012-07-02), XP055439182, Retrieved from the Internet: URL:http://cdn.intechopen.com/pdfs/2621

## Description

### Field of the invention

This invention relates to an antimicrobial composition. The invention more particularly relates to a personal care or cleansing composition e.g those for care or cleansing of hair or body which provides anti-microbial efficacy. It more particularly relates to a cleansing composition for hair and scalp comprising actives that interact to provide synergistic antimicrobial efficacy for anti-dandruff benefits.

### Background of the invention

The invention relates to an anti-microbial composition useful for cleansing of any body part but especially suitable for hair and scalp. Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts. To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g zinc pyrithione (ZPTO), octopirox (piroctone olamine), azole antimicrobials (e.g. climbazole), selenium sulfide and combinations thereof.

While the problem of dandruff is mitigated to a large extent through use of the above actives in such compositions, there is a need for enhancing the efficacy of these actives. The present inventors have found that the efficacy of one of the above actives, zinc pyrithione (ZPTO) can be enhanced when combined with habit modified crystals of zinc oxide. The habit modified crystals of zinc oxide are unique in that the ratio of photoluminescence peak intensity at 452 nm of the habit modified crystals of zinc oxide to that of zinc oxide is at least 5. The present inventors have achieved this by entrapping di or tri carboxylate in the crystal structure during the in situ preparation of the modified zinc oxide followed by calcining the particles at high temperatures. The inventors further found that conventional zinc oxide which is not habit modified as per the present invention do not provide the synergy along with ZPTO.

Zinc oxide is a potent bacteriostatic agent, i.e., it can arrest the growth of bacteria on a substrate or in a medium, but it does not have appreciable bactericidal activity.

Crystals of zinc oxide exist in the form of hexagonal rods of different sizes. Recent publications indicate that 3D structures of zinc oxide make up the largest group, which includes shapes like nanorods, needles, helixes, springs, rings, ribbons, tubes, belts, wires and combs, dandelion, flower snowflakes, coniferous and urchin-like. Zinc oxide is also obtainable in 2D structures, such as nanoplate/nanosheet and nanopellets. Examples of one-dimensional structures include nanorods and nanowires.

Zinc oxide has been the subject of several patents and research articles covering various forms, morphologies, crystal structures and applications thereof.

Zinc oxide (ZnO) generally exists as hexagonal rods in the Wurtzite crystal structure where O²⁻ and Zn²⁺ ions are tetrahedrally coordinated and stacked alternately along the Z-axis. Polarity of each crystal face of ZnO is different. In a hexagonal crystal structure, the two hexagonal faces are Zn⁺² rich, consequently they are positively charged, while the six rectangular faces are less positively charged due to the presence of Zn⁺² and O⁻² ions. This also means that the hexagonal faces are polar as compared to the rectangular faces.

Attempts were made in the past to restrict/ modulate the growth of specific faces of ZnO crystal by use of certain molecules, which when present in the reaction medium selectively bind to specific crystal faces resulting in changes in either the shape or the size of the crystal or both.

US2017209347 (Unilever) relates to a process for preparing an antimicrobial particulate composition and a personal care or hygiene composition comprising an antimicrobial particulate composition obtainable by the claimed process. It particularly relates to a process for preparing an antimicrobial metal nanoparticles (e.g. silver or copper) immobilized in an inorganic porous material preferably selected from zinc oxide, magnesium hydroxide or calcium carbonate.

US2009017303 (FAS Alliances) relates to a method for increasing photocatalytic activity of zinc oxide, which comprises preparing zinc oxide nanoplate crystals having a planar morphology on their (0001) crystal faces. In addition, the present invention relates to a process for synthesizing zinc oxide nanoplate crystals, a tooth whitening composition and a composition for degrading organic pollutants.

The above two publications relate to immobilization of metals in various particles like zinc oxide among others.

US2004191331 (P&G) discloses a composition comprising a particulate zinc material wherein the particulate zinc material has a crystallite size less than 600 A; and shampoo composition comprising an effective amount of a surfactant, an effective amount of a particulate zinc material, an effective amount of a metal salt of pyrithione, and an effective amount of a suspending agent wherein the particulate zinc material has a crystallite size less than 600 A.

The above publication discloses combination of conventional zinc oxide and zinc pyrithione.

Thus, to the knowledge of the present inventors, an antimicrobial composition comprising zinc pyrithione in combination with the habit modified crystals of zinc oxide disclosed in the present invention has not been known or published so far.

There is still need for newer and more efficacious compositions that synergistically enhance the antimicrobial efficacy of zinc pyrithione.

It is thus an obejct of the present invention to provide for a composition that exhibits enhanced antimicrobial activity especially antifungal activity in comparision to that obtained with zinc pyrithione alone.

### Summary of the invention

According to the first aspect of the present invention there is provided an antimicrobial composition comprising
(a) habit modified crystals of zinc oxide; and
(b) zinc pyrithione
wherein the ratio of photoluminescence peak intensity at 452 nm of the habit modified crystals of zinc oxide to that of zinc oxide is at least 5.

According to another aspect of the present invention there is provided a process to prepare a composition of the first aspect comprising the step of mixing zinc pyrithione with the habit modified crystals of zinc oxide; said habit modified crystals of zinc oxide are prepared by a process comprising the steps of:
(i) mixing aqueous solution of a di or tricarboxylate with aqueous solution of a non-hydrolysable water-soluble zinc salt;
(ii) adding a weak base thereto;
(iii) heating the reaction mixture of step (ii) to attain temperature of 60 to 90 °C;
(iv) filtering the contents of step (iii), washing the residue with water, and drying; and
(v) calcining the dried residue at 600 °C to 800 °C to obtain said habit modified crystals of zinc oxide.

Other aspects of the invention are discussed in detail in the following sections.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'A topical composition' or a 'skin care composition' as used herein, is meant to include a composition for topical application to the skin of mammals, especially humans. Such a composition could be of the leave-on or of the wash-off/ rinse-off type. By a leave-on composition is meant a composition that is applied to the desired skin surface and left on for a period of time (say from one minute to 24 hours) after which it may be wiped or rinsed off with water, usually during the regular course of personal washing. By a wash-off/ rinse off composition is meant a composition that is applied to the desired skin surface for a shorter period of time say of the order of seconds or minutes and usually contains sufficient surfactants that aids in cleaning the surface which may be rinsed off with copious amounts of water. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. The composition however comprises an emulsion or a gel as part of the cosmetically acceptable vehicle. "Skin" as used herein is meant to include skin on any part of the body e.g., neck, chest, back, arms, underarms, hands, legs, buttocks and scalp. When the product is used for the underarms it is usually called a deodorant product or a deo product. A class of deodorant product is the so called anti-perspirant (AP) product which contains an AP active which when applied to the axilla of an individual delivers anti-perspirancy and deodorancy benefits.

The composition of the present invention is preferably a wash off composition and is more preferably a personal cleansing composition. The composition is most preferably delivered as a shampoo, hair conditioner or a body wash composition.

According to a first aspect is disclosed comprising (a) habit modified crystals of zinc oxide; and (b)zinc pyrithione; wherein the ratio of photoluminescence peak intensity at 452 nm of the habit modified crystals of zinc oxide to that of zinc oxide is at least 5.

ZnO typically crystallizes as hexagonal rod-like crystals, having eight faces of which two are regular hexagon and six are rectangular. Habit modification is a process in which additives are introduced in the reaction medium during the formation of zinc oxide crystals which can alter the growth of the crystals thereby allowing and inhibiting growth in particular directions. This results in anisotropy and hence the morphology changes. The term "habit modification" is used commonly in crystal engineering.

By water soluble, as used herein, is meant that the solubility of the material in water at 25°C and atmospheric pressure is 0.1% by weight or more.

The morphology of zinc oxide crystals, the particle stacking and defects therein are modified by the selective use of di or tri carboxylates as crystal habit modifiers. Without wishing to be bound by theory, it is believed that these di and tri carboxylates may selectively bind to specific crystal faces and restrict the growth of crystal in specific directions. The two hexagonal faces of ZnO have relatively higher positive charged compared to the rectangular faces and hence the negatively charged carboxylates are more prone to bind to these hexagonal faces. Additionally, the entrapment of the carboxylates in the crystal matrix and formation of zinc salt of carboxylates cocrystals along with ZnO occurs. Elimination of the absorbed and chemically occluded carboxylate species results during calcination and results in the formation of defect lattice structures and morphological diversities in the crystals.

Crystalline materials normally crystallize from their solutions as having a shape that resembles their unit cell, the smallest unit of the material. Zinc oxide has the hexagonal unit cell and it typically crystallizes as hexagonal prismatic rods, with two hexagonal faces and six rectangular faces. Deliberate deviation of such natural morphology by playing with the crystallization is called crystal habit modification. It could be done by changing physical conditions like subjecting the system to different temperature, pressure, concentration or solvation conditions. Alternatively, one may employ traces of impurities that are known to preferentially adsorb on specific faces of the crystals thereby preventing growth on those faces. These impurities are called crystal habit modifiers. They often remain adsorbed on the faces of the crystals.

In the present invention, di or tri carboxylates are used as crystal habit modifiers that are believed to be responsible for deviation in the growth of zinc oxide from its natural hexagonal prismatic morphology to disk shaped and pseudospherical structures.

It has been found that mono or tetra carboxylates do not provide the enhanced functionality which the di or tri carboxylates deliver. Further it has been found that the aliphatic carboxylates are more effective than aromatic carboxylates. The aliphatic carboxylates that may be used in the present invention are preferably saturated.

The di or tri carboxylates that may be used are selected from one or more of citrate, oxalate or malonate. They are preferably alkali metal carboxylates, more preferably sodium carboxylates.

The structure of the preferred carboxylates are given below: 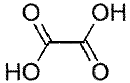 Oxalic acid 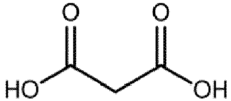 Malonic acid 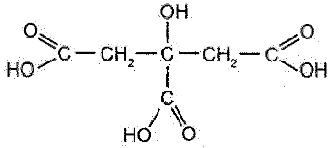 Citric acid

The habit modified crystals of zinc oxide of the invention are capable of industrial application *inter-alia* in the broad technical fields of home and personal care products. Non-limiting examples of specific fields include skin cleansing, hair care, surface hygiene and household care products.

Zinc oxide is used in several cosmetic compositions but the usual zinc oxide and at least some of its modified variants do not possess sufficient antimicrobial activity. It is a general practice to add known antimicrobial agents to top-up the antibacterial activity. The present invention provides a synergistic mixture of zinc pyrithione and the habit modified crystals of zinc oxide, which has significant antimicrobial efficacy even at low levels.

The habit modified crystals of zinc oxide for use in the composition of the present invention are preferably shaped like a disc. The thickness of each disc is preferably 100 nm to 250 nm. The average diameter of the discs is preferably in the range of 3 micron to 5 micron. The average size is measured by Malvern Multisizer^{®}, but any equivalent device may also be used. The zinc oxide particles are preferably in the form of discs of average size 2 µm to 4 µm.

Habit modified crystals of zinc oxide preferably has a formula ZnOₓ where x<1 and characterized by a photoluminescence peak at 452 nm Preferably x has a value in the range of 0.95 to 1. The composition of the invention preferably comprises 0.05 to 5%, more preferably 0.10 to 2.0%, further more preferably 0.2 to 1.0%, most preferably 0.3 to 0.5% by weight of the habit modified crystals of zinc oxide. The unique characteristics of the habit modified crystals of zinc oxide as per the invention is that the ratio of photoluminescence peak intensity at 452 nm of the habit modified crystals of zinc oxide to that of zinc oxide is at least 5, preferably at least 6, more preferably at least 6.5. Ideally the ratio is in the range of 6 to 9.

The composition of the invention comprises zinc pyrithione (ZPTO) which is shorthand for zinc 1-hydroxy-2-pyridinethione. The polyvalent metal salt of pyrithione is represented by the following general formula:

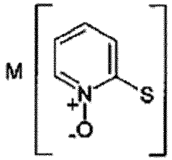

In the case of zinc pyrithione, M is the metal cation zinc.

Zinc pyrithione is preferably present in 0.01 to 10%, more preferably 0.01 to 5.0%, further more preferably from 0.05 to 2.0% based on weight of the composition. ZPTO is a particulate material. While the particle size is not critical to achieve the benefits of the present invention, the particle size of ZPTO is preferably from 0.25 to 8 micrometer, more preferably from 0.5 to 8.0 micrometer, and further more preferably from 1.0 to 7.5 micrometer. ZPTO is commercially available from Kolon Life Science Inc., Sino Lion (USA) Ltd, Lonza, and other suppliers.

The composition of the invention preferably additionally comprises a cosmetically acceptable carrier. The carrier is preferably chosen such that the composition of the invention can be delivered for use as a shampoo, a hair conditioner or a body wash. As per one aspect the cosmetically acceptable carrier is water or an aqueous solution. According to another preferred aspect, the carrier additionally comprises a surfactant. The cosmetically acceptable vehicle is such that the composition can be prepared as a shampoo, conditioner, body wash, hand wash or face wash product, cream, lotion, gel, powder, ointment, or a soap bar

According to a further preferred aspect of the present invention, the composition is either a shampoo, a hair conditioner, or a body wash product.

As per an especially preferred aspect of the invention, the composition is a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of the composition is preferably equal to or higher than 4.0, more preferably in the range of 5.0 to 10.0.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives.

Suspending agent, if included, will generally be present in a shampoo composition of the invention at levels of from 0.1 to 10%, preferably from 0.5 to 6%, more preferably from 0.5 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The composition of the invention is preferably aqueous based. It preferably comprises high amounts of water preferably from 70 to 95% by weight of the composition.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

The composition of the invention may be used for skin care e.g. body, hand or face wash. The antimicrobial composition may further comprise a surfactant. The preferred surfactants are nonionic surfactants, such as C₈-C₂₂, preferably C₈-C₁₆ fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Thus, in a highly preferred aspect, the antimicrobial compositions include the surfactant selected from the group of anionic surfactant, fatty acid amide, alkyl sulphate, linear alkyl benzene sulphonate, and combinations thereof.

When the surfactants are present, the antimicrobial composition preferably comprises 1 to 90% surfactant by weight of the composition

When surfactant is used, a particularly preferred surfactant is soap. Soap is a suitable surfactant for personal washing applications of the antimicrobial composition of the invention. The soap is preferably C₈-C₂₄ soap, more preferably C₁₀-C₂₀ soap and most preferably C₁₂-C₁₈ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also be used in other desired proportions.

When present, the soap, of the present is preferably present in an amount of 1 to 90%, preferably from 10 to 85%, more preferably 25 to 75% by weight of the composition.

Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, furthermore preferably at least 5% by weight of the composition. When water is the carrier, a preferred liquid composition comprises 10 to 99.8% by weight water.

According to another aspect of the present invention there is provided a process to prepare a composition of the invention comprising the step of mixing zinc pyrithione with said habit modified crystals of zinc oxide; said habit modified crystals of zinc oxide are prepared by a process comprising the steps of (i) mixing aqueous solution of a di or tricarboxylate with aqueous solution of a non-hydrolysable water-soluble zinc salt; (ii) adding a weak base thereto; (iii)heating the reaction mixture of step (ii) to attain temperature of 60 to 90 °C; (iv)filtering the contents of step (iii), washing the residue with water, and drying; and calcining the dried residue at 600 °C to 800 °C to obtain said habit modified crystals of zinc oxide.

It is preferred that the non-hydrolysable water-soluble zinc salt is a nitrate, sulphate, acetate or formate. The salt is preferably not a chloride salt.

The term "hydrolysable salt" means salts, which instantly (within five minutes of addition to water) forms either zinc oxide or basic oxy- hydroxides of zinc when added to water. Examples of hydrolysable salts of zinc include anhydrous zinc chloride, zinc complexes of substituted amino alcohols and sodium zincate. It is believed that hydrolysable salts form ZnO particles in the medium therefore, it is not possible to modulate the morphology/ defects/crystallinity when such salts are used.

It is preferred that the weak base is hexamethylenetetramine, urea or magnesium hydroxide. Other preferred weak bases include ammonia, pyridine, hydroxylamine and methylamine. It is preferred that the di or tri carboxylate is made to contact with the non-hydrolysable water soluble zinc salt, before the weak base is introduced into the reaction vessel. This ensures proper complexation of the zinc ions with the di or tri carboxylates. It is also preferred that the reaction mix, prior to introduction of the weak base, is heated to 40 °C to 60 °C for about 30 minutes. On the other hand, if the base is contacted with the zinc salt prior to addition of the carboxylate, it results in the formation of very small seed crystals of hexagonal zinc oxide particles in the medium. The resultant product may have significantly lower photoluminescence and photo-catalytic activity.

Strong bases, such as sodium hydroxide, should not be used in the process because there is likelihood of faster precipitation.

Upon addition of the weak base, the reaction mixture of step (ii) is heated to attain a temperature of 60 to 90 °C. In a preferred aspect of the method, the heated reaction mixture of step (iii) is autoclaved under pressure of 4 to 6 bar in an inert atmosphere. Autoclaving leads to a more efficacious product as compared to its non-autoclaved counterpart.

The contents of step (iii) is filtered by any suitable means. The residue is washed with water and thereafter it is dried.

The dried powder from step (iv) is then calcined at 600 °C to 800 °C. Calcination is also used to imply a thermal treatment process in the absence or limited supply of air or oxygen applied to ores and other solid materials to bring about a thermal decomposition, phase transition, or removal of a volatile fraction. Calcination normally takes place at temperatures below the melting point of a given material.

Also disclosed in accordance with this invention is non-therapeutic use of the composition for delivering anti-microbial benefits.

The following non-limiting examples further illustrate preferred embodiments of the invention. All percentages referred to in the examples and throughout this specification are based on total weight unless otherwise indicated.

### Examples

Example A: 0.30 gram of sodium citrate was added to 50 ml distilled water. The contents were stirred well until a clear solution was obtained. To this solution, an aqueous solution of 3 g zinc nitrate hexahydrate was added. The solution was heated at 40 °C for 15 minutes. 1.5 g of hexamine was dissolved in 50 ml of water. Hexamine solution was added to the mixture. The contents were transferred to a stoppered hydrothermal bottle and heated in an air oven at 90 °C for 24 hours. under pressure of 5.0 bar in an atmosphere of nitrogen. The habit modified crystals of zinc oxide that were formed, were filtered, washed with water and air-dried. The dried powder was calcined at 500 °C for two hours to prepare sample of Example A.

Sodium citrate is a salt of tricarboxylic acid.

Example B: The sample was zinc pyrithione sourced from sigma-aldrich.

Example C: ZnO commercially available from Merck as EMSURE^{®} ACS, Reagent brand calcined for two hours at 700 °C+ ZPTO at a weight ratio of 1:2.

Example 1: Example A + Example B at a weight ratio of 1:2

Example 2: Example A except that sodium oxalate was used instead of sodium citrate + Example B at weight ratio of 1:2. Sodium oxalate is a salt of dicarboxylic acid.

Example D: Example A except that sodium acetate was used instead of sodium citrate + Example B at weight ratio of 1:2. Sodium oxalate is a salt of dicarboxylic acid.

Example E: Example A except that sodium salt of ethylene diamine tetraacetic acid (EDTA) was used instead of sodium citrate + Example B at weight ratio of 1:2. EDTA is a tetra carboxylic acid.

The above samples were subjected to their anti fungal efficacy on *M. furfur* as described hereinafter.

### Antifungal activity:

M. furfur ATCC 14521 strain was revived from glycerol stock on Modified Dixon agar. Plate was at 30°C for 72 hrs. Approximately 72 hrs prior to testing, the plate culture was inoculated onto the surface of another sterile Modified Dixon agar plate and incubated at 30°C for approximately 72 hrs. From second subculture, the optical density of culture is adjusted at 620 nm to give cell strength of 10⁷ cfu/ml. The culture was further diluted for 100 times to achieve 10⁵ cfu/ml in Pityrosporum broth.

10 mg of each sample (the sample is particulate hence it has to be thoroughly mixed such as vortex/ sonication) was weighed into a sterile 1.5 ml Eppendorf tube. 1000 µL of M. furfur inocula were added to the above tubes and a blank sterile 1.5 ml Eppendorf tube (no particles as control), respectively so that the load in each tube is 10⁵ cells/ml.

All the samples were kept shaken @120 RPM in a shaker incubator at 32°C. After 24 hours incubation, 10 µL of the samples were withdrawn from the respective 1.5 ml Eppendorf tubes and diluted with 990 µL of Pityrosporum broth. This represents 10⁻² dilution. Serial dilution was performed from the above 24 hours inocula and plated 100 µL of dilution onto modified Dixon plate. Plate dilution 10⁻², 10⁻³, 10⁻⁴ and 10⁻⁵. All the plates were kept for incubation at 32°C for 24 - 72 hrs. Plate count for all the plates were done.

The data on the antifungal activity in terms of log reduction is summarized in Table - 1 below:

**Table - 1:**

| Example | Sample | Contact time, hr | Log reduction |
|---|---|---|---|
| A | Calcined sample of crystals of ZnO habit modified with sodium citrate | 24 | 1.23 |
| B | ZPTO | 24 | 1.58 |
| C | Calcined sample of commercial ZnO + ZPTO | 24 | 1.87 |
| D | Calcined sample of crystals of ZnO habit modified with sodium acetate + ZPTO (weight ratio 1:2) | 24 | 1.53 |
| 1 | Calcined sample of crystals of ZnO habit modified with sodium citrate + ZPTO (weight ratio 1:2) | 24 | 2.77 |
| 2 | Calcined sample of crystals of ZnO habit modified with sodium oxalate + ZPTO (weight ratio 1:2) | 24 | 2.48 |
| E | Calcined sample of crystals of ZnO habit modified with sodium EDTA + ZPTO (weight ratio 1:2) | 24 | 1.71 |

| | | | |
|---|---|---|---|
| The data in Table - 1 indicates that a composition as per the invention (Example 1 and 2) provide vastly superior efficacy as compared to those outside the invention (Examples A to E). Further, Example 1 indicates synergistic antimicrobial efficacy as compared to results obtained in Example A and B. | | | |

Example F: Example A except that sodium salt of trimesic acid was used instead of sodium citrate + Example B at weight ratio of 1:2. Sodium salt of trimesic acid has the structure as given below. Trimesic acid is a triaromatic acid.

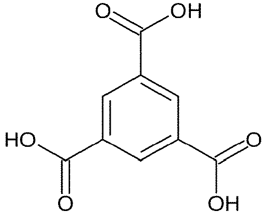

Example G: Example A except that sodium phthalate was used instead of sodium citrate + Example B at weight ratio of 1:2. Sodium phthalate has the structure as given below. Phthalic acid is a diaromatic acid.

Example 3: Example A except that sodium malonate was used instead of sodium citrate + Example B at weight ratio of 1:2. Sodium malonate is a dialiphatic acid.

The samples of Examples F, G and 3 were analysed for anti fungal efficacy as described hereinabove. The results on log reduction together with results for examples A, B and 1 (for comparison) are summarized in Table 2 below.

**Table - 2:**

| Example | Sample | Contact time, hr | Log reduction |
|---|---|---|---|
| A | Calcined sample of crystals of ZnO habit modified with sodium citrate | 24 | 1.23 |
| B | ZPTO | 24 | 1.58 |
| 1 | Calcined sample of crystals of ZnO habit modified with sodium citrate + ZPTO (weight ratio 1:2) | 24 | 2.77 |
| F | Calcined sample of crystals of ZnO habit modified with sodium salt of trimesic acid + ZPTO (weight ratio 1:2) | 24 | 1.65 |
| G | Calcined sample of crystals of ZnO habit modified with sodium phthalate + ZPTO (weight ratio 1:2) | 24 | 1.71 |
| 3 | Calcined sample of crystals of ZnO habit modified with sodium malonate + ZPTO (weight ratio 1:2) | 24 | 2.73 |

| | | | |
|---|---|---|---|
| The data in Table - 2 indicates that a composition as per the invention (Example 1 and 3) which utilize di or tri aliphatic carboxylates provide vastly superior efficacy as compared to samples where salt of aromatic acid (Examples F and G) were used. | | | |

Photoluminescence spectra of various samples of calcined zinc oxide at 452 nm at 0.5 mg/ml concentration was measured and the data in intensity (a.u) is summarized in the table-3 below:

**Table 3**

| Example | Sample | Photoluminescence intensity (a.u) at 452 nm | Ratio |
|---|---|---|---|
| H | Calcined sample of commercial ZnO | 2 | - |
| I | Calcined sample of crystals of ZnO habit modified with sodium acetate | 6 | 3 |
| 4 | Calcined sample of crystals of ZnO habit modified with sodium oxalate | 13 | 6.5 |
| 5 | Calcined sample of crystals of ZnO habit modified with sodium citrate | 17 | 8.5 |
| J | Calcined sample of crystals of ZnO habit modified with sodium salt of EDTA | 5 | 2.5 |
| K | Calcined sample of crystals of ZnO habit modified with sodium salt of trimesic acid | 7 | 3.5 |
| 6 | Calcined sample of crystals of ZnO habit modified with sodium malonate | 16 | 8 |
| L | Calcined sample of crystals of ZnO habit modified with sodium phthalate | 6 | 3 |

| | | | |
|---|---|---|---|
| "Ratio" in table above is the ratio of photoluminescence peak intensity at 452 nm of the habit modified crystals of zinc oxide to that of zinc oxide. | | | |

The data in the above tables (1 to 3) indicates that habit modified zinc oxide where the ratio of photoluminescence intensity of the modified samples to that of commercial zinc oxide is more than 5 (Examples 4 to 6) provide much higher antimicrobial activity when combined with ZPTO as compared to samples where the ratio is less than 5 (Examples H to L).

## Claims

1. An antimicrobial composition comprising
(a) habit modified crystals of zinc oxide; and
(b) zinc pyrithione
wherein the ratio of photoluminescence peak intensity at 452 nm of the habit modified crystals of zinc oxide to that of zinc oxide is at least 5.

2. A composition as claimed in claim 1 comprising 0.01 to 10%, preferably 0.01 to 5%, more preferably 0.05 to 2% zinc pyrithione by weight of the composition.

3. A composition as claimed in claim 1 or 2 comprising 0.05 to 5%, of said habit modified crystals of zinc oxide.

4. A composition as claimed in any one of the preceding claims additionally comprising a cosmetically acceptable carrier.

5. A composition as claimed in any one of the preceding claims wherein said composition is a personal cleansing composition.

6. A composition as claimed in claim 5 wherein said composition is a shampoo, hair conditioner or a body wash composition.

7. A composition as claimed in any one of the preceding claims wherein said habit modified crystal of zinc oxide has a formula ZnOₓ where x<1.

8. A process to prepare a composition as claimed in claim 1 comprising the step of mixing zinc pyrithione with said habit modified crystals of zinc oxide; said habit modified crystals of zinc oxide are prepared by a process comprising the steps of:
(i) mixing aqueous solution of a di or tricarboxylate with aqueous solution of a non-hydrolysable water-soluble zinc salt;
(ii) adding a weak base thereto;
(iii) heating the reaction mixture of step (ii) to attain temperature of 60 to 90 °C;
(iv) filtering the contents of step (iii), washing the residue with water, and drying; and
(v) calcining the dried residue at 600 to 800 °C to obtain said habit modified crystals of zinc oxide.

9. A process as claimed in claim 8 wherein heated reaction mixture of step (iii) is autoclaved under pressure of 4 to 6 bar in an inert atmosphere.

10. A process as claimed in claim 8 or 9 wherein said di or tri carboxylate is saturated.

11. A process as claimed in any one of the preceding claims 8 to 10 wherein said di or tri carboxylate is aliphatic.

12. A process as claimed in any one of the preceding claims 8 to 11 wherein said di or tri carboxylate is selected from citrate, oxalate or malonate.

13. Non-therapeutic use a composition as claimed in any one of the preceding claims 1-7 as an antimicrobial composition.

## Patentansprüche

1. Antimikrobielle Zusammensetzung, umfassend
(a) Habitus-modifizierte Kristalle von Zinkoxid und
(b) Zink-Pyrithion,
wobei das Verhältnis der Photolumineszenz-Peakintensität bei 452 nm der Habitus-modifizierten Kristalle von Zinkoxid zu der von Zinkoxid mindestens 5 beträgt.

2. Zusammensetzung, wie im Anspruch 1 beansprucht, umfassend 0,01 bis 10%, vorzugsweise 0,01 bis 5%, bevorzugter 0,05 bis 2% Zink-Pyrithion der Zusammensetzung.

3. Zusammensetzung, wie im Anspruch 1 oder 2 beansprucht, umfassend 0,05 bis 5% der Habitus-modifizierten Kristalle von Zinkoxid.

4. Zusammensetzung, wie in irgendeinem der vorhergehenden Ansprüche beansprucht, zusätzlich umfassend einen kosmetisch verträglichen Träger.

5. Zusammensetzung, wie in irgendeinem der vorhergehenden Ansprüche beansprucht, wobei die Zusammensetzung eine persönliche Reinigungszusammensetzung ist.

6. Zusammensetzung, wie im Anspruch 5 beansprucht, wobei die Zusammensetzung ein Shampoo, ein Haarspülmittel oder eine Körperwaschzusammensetzung darstellt.

7. Zusammensetzung, wie in irgendeinem der vorhergehenden Ansprüche beansprucht, wobei das Habitus-modifizierte Kristall von Zinkoxid eine Formel ZnOₓ aufweist, wobei x < 1 ist.

8. Verfahren zur Herstellung einer Zusammensetzung, wie im Anspruch 1 beansprucht, umfassend den Schritt des Mischens von Zink-Pyrithion mit den Habitus-modifizierten Kristallen von Zinkoxid, wobei die Habitus-modifizierten Kristalle von Zinkoxid durch ein Verfahren hergestellt werden, das die Schritte umfasst:
(i) Mischen einer wässrigen Lösung eines Di- oder Tricarboxylats mit einer wässrigen Lösung eines nicht-hydrolysierbaren wasserlöslichen Zinksalzes;
(ii) Dazugeben einer schwachen Base;
(iii) Erhitzen der Reaktionsmischung vom Schritt (ii), um eine Temperatur von 60 bis 90°C zu erreichen;
(iv) Filtern des Inhalts vom Schritt (iii), Waschen des Rückstands mit Wasser und Trocknen; und
(v) Calcinieren des getrockneten Rückstands bei 600 bis 800°C, um Habitus-modifizierte Kristalle von Zinkoxid zu erhalten.

9. Verfahren, wie im Anspruch 8 beansprucht, wobei die erhitzte Reaktionsmischung vom Schritt (iii) unter einem Druck von 4 bis 6 bar in einer inerten Atmosphäre autoklaviert wird.

10. Verfahren, wie im Anspruch 8 oder 9 beansprucht, wobei das Di- oder Tricarboxylat gesättigt ist.

11. Verfahren, wie in irgendeinem der vorhergehenden Ansprüche 8 bis 10 beansprucht, wobei das Di- oder Tricarboxylat aliphatisch ist.

12. Verfahren, wie in irgendeinem der vorhergehenden Ansprüche 8 bis 11 beansprucht, wobei das Di- oder Tricarboxylat unter Citrat, Oxalat oder Malonat ausgewählt ist.

13. Nicht-therapeutische Verwendung einer Zusammensetzung, wie in irgendeinem der vorhergehenden Ansprüche 1-7 beansprucht, als antimikrobielle Zusammensetzung.

## Revendications

1. Composition antimicrobienne comprenant
(a) des cristaux à habitus modifié d'oxyde de zinc ; et
(b) de la pyrithione de zinc
dans laquelle le rapport d'intensité de pic de photoluminescence à 452 nm des cristaux à habitus modifié d'oxyde de zinc à celle d'oxyde de zinc est d'au moins 5.

2. Composition selon la revendication 1 comprenant de 0,01 à 10 %, de préférence de 0,01 à 5 %, encore mieux de 0,05 à 2 % de pyrithione de zinc en masse de la composition.

3. Composition selon la revendication 1 ou 2 comprenant de 0,05 à 5 %, desdits cristaux à habitus modifié d'oxyde de zinc.

4. Composition selon l'une quelconque des revendications précédentes comprenant de plus un support cosmétiquement acceptable.

5. Composition selon l'une quelconque des revendications précédentes, dans laquelle ladite composition est une composition nettoyante pour la personne.

6. Composition selon la revendication 5, dans laquelle ladite composition est un shampoing, après-shampoing ou une composition pour le lavage du corps.

7. Composition selon l'une quelconque des revendications précédentes, dans laquelle ledit cristal à habitus modifié d'oxyde de zinc présente une formule ZnOₓ où x < 1.

8. Procédé pour la préparation d'une composition selon la revendication 1 comprenant l'étape de mélange de pyrithione de zinc avec lesdits cristaux à habitus modifié d'oxyde de zinc ; lesdits cristaux à habitus modifié d'oxyde de zinc sont préparés par un procédé comprenant les étapes de :
(i) mélange de solution aqueuse d'un di ou tricarboxylate avec une solution aqueuse d'un sel de zinc soluble dans l'eau non hydrolysable ;
(ii) addition d'une base faible à celle-ci ;
(iii) chauffage du mélange réactionnel de l'étape (ii) pour atteindre une température de 60 à 90°C ;
(iv) filtration des contenus de l'étape (iii), lavage du résidu avec de l'eau, et séchage ; et
(v) calcination du résidu séché à de 600 à 800°C pour obtenir lesdits cristaux à habitus modifié d'oxyde de zinc.

9. Procédé selon la revendication 8, dans lequel le mélange réactionnel chauffé de l'étape (iii) est autoclavé sous une pression de 4 à 6 bars dans une atmosphère inerte.

10. Procédé selon la revendication 8 ou 9, dans lequel ledit di ou tricarboxylate est saturé.

11. Procédé selon l'une quelconque des revendications 8 à 10 précédentes, dans lequel ledit di ou tricarboxylate est aliphatique.

12. Procédé selon l'une quelconque des revendications 8 à 11 précédentes, dans lequel ledit di ou tricarboxylate est choisi parmi un citrate, oxalate ou malonate.

13. Utilisation non-thérapeutique d'une composition selon l'une quelconque des revendications 1-7 précédentes comme une composition antimicrobienne.
